# EUROPEAN PATENT APPLICATION

(11) **EP 4 806 423 A1**
(43) Date of publication of application: **16.09.2026**
(21) Application number: 24913827.2
(22) Date of filing: 19.12.2024
(51) Int. Cl.: A61K 9/00, A61K 9/08, A61K 31/58, A61P 17/14

(54) **NOVEL LONG-ACTING INJECTABLE COMPOSITION COMPRISING FINASTERIDE FOR PREVENTION OR TREATMENT OF ANDROGENETIC ALOPECIA**

(30) Priority: 27.12.2023 US 202363614964 P; 11.12.2024 US 202418976930
(71) Applicant: Inventage Lab Inc., Gyeonggi-do 13403 (KR)
(72) Inventor: KIM, Ju Hee, Seongnam-si, Gyeonggi-do 13494 (KR)
(74) Representative: BCKIP Part mbB
(86) International application number: PCT/KR2024/097171
(87) International publication number: WO 2025/144023

(57) **Abstract**

The present invention relates to a novel long-acting injectable composition comprising finasteride for the prevention or treatment of androgenetic alopecia. The composition inhibits type II 5α-reductase with a single injection lasting over one month, thereby suppressing the conversion of testosterone to DHT and reducing androgen-mediated follicular miniaturization. In addition, after injection into the body, the average elimination half-life (T_{1/2e1}) of finasteride can be increased to enhance stability and provide more predictable and uniform therapeutic effects compared to oral formulations.

## Description

### Technical Field

The present invention relates to a novel long-acting injectable composition for preventing or treating androgenetic alopecia containing finasteride.

### Background Art

In androgenetic alopecia, a phenomenon occurs in which healthy hair gradually becomes thinner and shorter, and the hair becomes weaker and breaks more easily. This phenomenon is called miniaturization. Hair follicles that undergo miniaturization eventually become thinner, are less visible, and produce short and fine hairs, which leads to hair loss. Accordingly, many studies have been reported recently on the prevention and treatment of hair loss by inhibition of male hormone activity.

Testosterone is converted into the active male hormone dihydrotestosterone (DHT) by 5α-reductase, and the activated dihydrotestosterone binds to the androgen receptor, thereby delaying protein synthesis in hair follicle cells, thus shortening the growth period of hair follicles, and causes hair follicle atrophy, resulting in hair loss.

In addition, during androgenetic alopecia, sebum is excessively produced, and as a result, hair loss accompanied by inflammation may appear on the scalp (Dennis A. Holt, et. al,. 1990).

Finasteride (Fi) and dutasteride, which are 5α-reductase inhibitors, are known to be effective in treating alopecia by inhibiting the 5α-reductase-mediated conversion of testosterone into its active metabolite, dihydrotestosterone (DHT).

Finasteride, a 5α-reductase inhibitor currently on the market, is sold as an oral medication that must be taken daily, and thus finasteride should be taken daily to prevent hair loss. If taking finasteride is stopped, the 5α-reductase inhibitory effect will stop, and hair loss symptoms may reappear.

To solve the above problem, there is a need to develop a long-acting formulation of finasteride that can exhibit the effect of preventing and treating hair loss for one month or more with a single dose.

### [Prior Art Documents]

### [Patent Documents]

KR 10-2021-0129565 A1

### DISCLOSURE

### Technical Problem

An object of the present invention is to provide a novel long-acting injectable composition for preventing or treating androgenetic alopecia containing finasteride.

Another object of the present invention is to provide a novel long-acting injectable composition for preventing or treating androgenetic alopecia containing finasteride, which is capable of inhibiting type II 5α-reductase for one month or more with a single injection, thereby limiting the conversion of testosterone to DHT and inhibiting androgen-mediated hair follicle miniaturization.

Another object of the present invention is to provide a novel long-acting injectable composition for preventing or treating androgenetic alopecia containing finasteride, which may increase the mean elimination half-life (t_{1/2el}) of finasteride after *in vivo* injection, thereby increasing the stability of finasteride, and exhibiting a more predictable and uniform therapeutic effect compared to oral dosage forms.

### Technical Solution

To achieve the above-described objects, the present invention may provide a novel long-acting injectable composition for preventing or treating androgenetic alopecia, which contains finasteride at a dose of 10 mg to 40 mg and inhibits androgen-mediated hair follicle miniaturization by finasteride for one month or more.

In addition, the injectable composition may be a subcutaneous (SC) injection formulation.

In addition, the mean elimination half-life (t_{1/2el}) of finasteride *in vivo* following administration of the injectable composition may be 100 to 160 hours.

In addition, the first-order absorption rate constant (Ka) of finasteride following administration of the injectable composition may be 0.0005 to 0.0015 h⁻¹.

In addition, the volume of distribution of finasteride in central compartment (tvV) following administration of the injectable composition may be 500 to 1,500 L.

In addition, the volume of distribution of finasteride in peripheral compartment (tvV2) following administration of the injectable composition may be 0.1 to 0.5 L.

In addition, the clearance of finasteride from central compartment (tvCL) following administration of the injectable composition may be 5 to 15 L/h.

In addition, the clearance of finasteride between central compartment and peripheral compartment (tvCL2) following administration of the injectable composition may be 0.005 to 0.015 L/h.

In addition, the maximum plasma concentration (Cₘₐₓ) of finasteride following administration of the injectable composition may be 500 to 3,000 pg/mL.

In addition, the steady-state average concentration (C_{ss, avg}) of finasteride following administration of the injectable composition may be 1,000 to 6,000 pg/mL.

In addition, the AUC at 0 hour to 672 hours (ACU₀₋₆₇₂ₕ) of finasteride following administration of the injectable composition may be 500,000 to 2,000,000 hr*pg/mL.

In addition, the steady-state AUC (AUCss) of finasteride following administration of the injectable composition may be 1,000,000 to 4,000,000 hr*pg/mL.

### Advantageous Effects

The injectable composition according to the present invention is capable of inhibiting type II 5α-reductase for one month or more with a single injection, thereby limiting the conversion of testosterone to DHT and inhibiting androgen-mediated hair follicle miniaturization.

In addition, the injectable composition according to the present invention may increase the mean elimination half-life (t_{1/2el}) of finasteride after *in vivo* injection, thereby increasing the stability of finasteride, and exhibiting a more predictable and uniform therapeutic effect compared to oral dosage forms.

### Brief Description of Drawings

FIG. 1 is a flow chart showing selection and assignment of subjects according to one embodiment of the present invention.
FIG. 2 is a schematic diagram showing a study design according to one embodiment of the present invention.

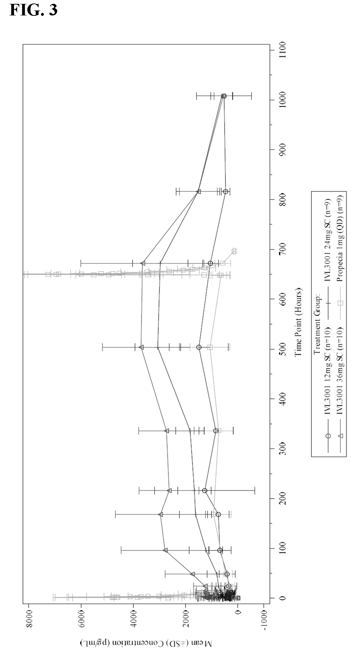

FIG. 3 shows mean blood concentration-time curves of IVL3001 and oral finasteride formulations according to one embodiment of the present invention.
FIG. 4 relates to estimated pharmacodynamic parameters in a validation model according to one embodiment of the present invention.
FIG. 5 shows the results of baseline-corrected DHT median pharmacodynamic measurement according to one embodiment of the present invention.
FIG. 6 shows observed and predicted plasma DHT concentrations following a single administration of IVL3001 and oral finasteride formulations according to one embodiment of the present invention.

### Best Mode

The present invention relates to a novel long-acting injectable composition for preventing or treating androgenetic alopecia, which contains finasteride at a dose of 10 mg to 40 mg and inhibits androgen-mediated hair follicle miniaturization by finasteride for one month or more.

### Mode for Invention

Hereinafter, embodiments of the present invention will be described in detail so that those skilled in the art can easily implement the present invention. However, the present invention may be embodied in various different forms and is not limited to the embodiments described herein.

Androgenetic alopecia (AGA) or male pattern hair loss (MPHL) is a genetic disorder that affects approximately 50% of men over 50 years of age and a small percentage of women, but it remains an important genetic disorder in women.

AGA is a common form of hair loss in both adult men and women. AGA patients typically present with progressive thinning and shortening of the hair in the affected area. In men, AGA is typically characterized by a patterned hair loss with a receding hairline and baldness at the crown. However, in women, AGA is characterized by overall thinning of hair. AGA is a source of psychological stress for both men and women, and is likely to be even more distressing for women.

Furthermore, the prognosis of AGA can significantly impair the quality of life of affected individuals. AGA is a disorder caused by a combination of genetic predisposition and androgen influence on the hair follicles of the scalp.

There are currently two drugs approved by the U.S. Food and Drug Administration (FDA) for the treatment of AGA, one of which is finasteride. Men predisposed to AGA have a high rate of conversion of testosterone to dihydrotestosterone (DHT) within the hair follicle, a process involving type II 5a-reductase.

Finasteride is a synthetic anti-androgen that inhibits type II 5a-reductase, thereby limiting the conversion of testosterone to DHT and inhibiting androgen-mediated hair follicle miniaturization, and thus may have a positive effect on AGA patients. Finasteride has been tested at various doses, taking into account its short terminal half-life of 4 to 8 hours. Previous reports have recommended that taking 1 mg of finasteride daily is most effective in treating men with AGA.

In previous clinical studies using 1 mg finasteride tablets, the bioavailability of finasteride was about 65%. In addition, the half-life of finasteride affects its long-term effect, and thus finasteride should be taken daily to maintain the necessary therapeutic drug concentration. Therefore, the inconvenience of having to take finasteride daily is considered a significant disadvantage of existing finasteride dosage forms.

Long-acting injectables (LAI) may offer advantages over oral administration in a variety of medical contexts. Therefore, to overcome the shortcomings of oral finasteride, the inventors of the present invention developed a LAI formulation of microparticles containing finasteride and administered the same by subcutaneous (SC) injection to deliver finasteride into the body. The present invention demonstrated that subcutaneous injection of the injectable composition of the present invention is more effective in preventing testosterone-induced hair loss in animal models compared to daily oral administration of finasteride. Specifically, finasteride-loaded microspheres stimulated hair follicle cell growth signaling in mouse skin by increasing the activities of phosphoinositide 3-kinase/protein kinase B and Wnt/β-catenin. In addition, the microspheres suppressed the apoptosis of hair follicle cells by down-regulating the expression of transforming growth factor-β2 and caspase-3.

However, in the results of the above-described animal experiment, not only was it impossible to determine the appropriate administration dose to maintain the effect of preventing testosterone-induced hair loss for one month or more, but it was also impossible to determine the maximum plasma concentration of finasteride when administered to humans, not animal models.

The present invention relates to a dose range capable of exhibiting the effect of releasing finasteride in sustained manner for one month or more, and to specific characteristics compared to existing oral dosage forms when administered within the dose range.

Specifically, the dose of finasteride may be 10 mg to 40 mg, at which androgen-mediated hair follicle miniaturization may be inhibited by finasteride for one month or more. Specifically, the dose of finasteride to maintain the preventive and therapeutic effect of finasteride against testosterone-induced hair loss for one month or more may be 10 mg to 40 mg, 11 mg to 39 mg, 12 mg to 38 mg, 12 mg, 24 mg, or 38 mg, but is not limited to the above dose, and any dose that does not cause initial over-release, can slow down the absorption rate in the body, can increase the half-life of finasteride, enables more efficient distribution throughout the body after being absorbed in the body, and can slow down the rate of elimination from the body may be used without limitation.

Pharmacokinetic parameters related to absorption are kₐ and F. When a drug is taken with water, it enters the stomach and goes down to the small intestine, where it undergoes an absorption process. The first step in the absorption process is that the entered drug in the form of a tablet or capsule breaks down and dissolves in the digestive fluid, and then the drug molecules dissolved therein pass through the intestinal epithelial cells. The first-order absorption rate constant, kₐ, is related to how quickly the disintegration and dissolution process that occurs in the digestive tract occurs. A drug that dissolves faster and more quickly passes through the intestinal epithelial cells into the bloodstream has a higher kₐ.

In the injectable composition of the present invention, the first-order absorption rate constant (Ka) of finasteride following administration of the injectable composition may be 0.0005 to 0.0015 h⁻¹, 0.0006 to 0.0014 h⁻¹, 0.0007 to 0.0013 h⁻¹, 0.0008 to 0.0012 h⁻¹, 0.0009 to 0.0011 h⁻¹, or 0.001 h⁻¹, without being limited thereto. When finasteride is administered into the body using the injectable composition of the present invention, it exhibits a first-order absorption rate constant which is absolutely smaller than that of a conventional oral formulation, and is absorbed slowly, suggesting that it exhibits an effect for one month or more with a single injection.

In the early 20^{th} century, with the advent of technologies for measuring plasma drug concentrations, it was discovered that plasma drug concentrations measured after intravenous infusion exhibited a concave curve where the plasma drug concentration initially decreased rapidly over time and then gradually decreased. When drawn in Iogarithmic concentration units, the concentration curve changes to a straight line, so it can be written as an equation such as In(C) = In(C(0))-kt. Here, C(0) is a straight line with a concentration at time 0 and a slope of -k according to time t indicated on the x-axis. If the exponents are taken on both sides of this equation to remove the logarithmic unit and the equation is transformed into a differential equation, an equation such as dC/dt = -kC is obtained. This simple form of differential equation means that every moment, the degree of change in concentration over time (dC/dt) is equal to the power of concentration 1 (C = C¹) and is proportional to (-kC). Since the direction of the change is decreasing over time, "-" is added to the proportional constant k. Thus, it is said that the rate of change of a value is proportional to its value itself (to the first power), following first- order kinetics. Drugs that are eliminated according to the first-order kinetics have a characteristic "half-life" (t_{1/2}). Half-life is the time for plasma drug concentrations to be reduced by half, and for drugs that are eliminated according to the first-order kinetics, each drug has a certain half-life (t_{1/2}).

In the case of finasteride, which is usually provided as an oral formulation, the *in vivo* mean half-life is only about 6.4 hours, but it was confirmed that the mean elimination half-life (t_{1/2el}) of finasteride *in vivo* following administration of the injectable composition of the present invention was 100 to 160 hours, which was greatly different from that of the oral formulation. Due to this difference in half-life, when finasteride is provided as a formulation disclosed in the present invention, it may have increased stability and exhibit a uniform therapeutic effect through sustained and controlled release, compared to when finasteride is provided as an oral formulation.

When the injectable composition of the present invention is administered to an actual human body, it moves along the blood vessels and is distributed in the liver, the kidneys, and the rest of the body. More specifically, the injectable composition is distributed in blood vessels (plasma) and organs such as liver or kidneys, which have a lot of blood flow and where drugs are easily distributed. These tissues are called the central compartment. The rest of the body where the drug is distributed later is called the peripheral compartment. The phase in which the plasma drug concentration drops rapidly immediately after injection is called the distribution phase. The next phase in which the concentration drops with a gentle slope after the equilibrium between two fractions is reached to some extent is called the elimination phase, because the plasma drug concentration is believed to drop mainly due to the phenomenon in which the drug is eliminated from the body. Even in the distribution phase, the mechanism by which the drug is eliminated from the body is working, but plasma (part of the central compartment) contributes more to the drop in the drug concentration, because the effect of the mechanism of movement (diffusion) to the peripheral compartment is believed to be more pronounced than the mechanism of elimination from the body.

The volume of distribution of finasteride in central compartment (tvV) following administration of the injectable composition of the present invention may be 500 to 1,500 L, 600 to 1,400 L, 700 to 1,300 L, 800 to 1,200 L, or 900 to 1,100 L. A large volume of distribution in the central compartment means that, even when the same amount of finasteride is administered, it is distributed more in the tissue than in the plasma. This means that, when the injectable composition of the present invention is used, the amount of finasteride that can bind to the tissue surface or penetrate the cell membrane is greater compared to when an oral formulation of finasteride is used.

On the other hand, the volume of distribution of finasteride in peripheral compartment (tvV2) following administration of the injectable composition of the present invention may be 0.1 to 0.5 L, 0.11 to 0.4 L, 0.12 to 0.3 L, 0.14 to 0.3 L, 0.15 to 0.3 L, 0.16 to 0.3 L, 0.17 to 0.3 L, 0.18 to 0.3 L, 0.19 to 0.3 L, 0.20 to 0.3 L, 0.20 to 0.29 L, 0.20 to 0.28 L, 0.20 to 0.27 L, 0.20 to 0.26 L, 0.20 to 0.25 L, 0.20 to 0.24 L, 0.20 to 0.23 L, 0.20 to 0.22 L, or 0.21 to 0.22 L. As described above, as finasteride in the injectable composition of the present invention has a large volume of distribution in the central compartment and a small volume of distribution in the peripheral compartment, it can be seen that finasteride is easily distributed around the area where blood flow is abundant.

In addition, the clearance of finasteride from central compartment (tvCL) following administration of the injectable composition may be 5 to 15 L/h, 6 to 14 L/h, 7 to 13 L/h, 8 to 12 L/h, or 9 to 11, and the clearance of finasteride between central compartment and peripheral compartment (tvCL2) following administration of the injectable composition may be 0.005 to 0.015 L/h, 0.006 to 0.014 L/h, 0.007 to 0.013 L/h, 0.008 to 0.012 L/h, or 0.009 to 0.011 L/h. These values are reduced values compared to when the oral formulation is used. This means that finasteride administered *in vivo* can exert its effect while remaining for a long time.

In addition, the maximum plasma concentration (Cₘₐₓ) of finasteride following administration of the injectable composition may be 500 to 3,000 pg/mL, 600 to 2,900 pg/mL, 700 to 2,800 pg/mL, 800 to 2,800, or 900 to 2,800 pg/mL. The maximum plasma concentration varies depending on the dose of finasteride contained in the injectable composition of the present invention. When the dose is 12 mg/4 weeks, the maximum plasma concentration (Cₘₐₓ) may be 500 to 1,300 pg/mL, 600 to 1,200 pg/mL, 700 to 1,100 pg/mL, or 800 to 1,000 pg/mL, and when the dose is 24 mg/4 weeks, the maximum plasma concentration (Cₘₐₓ) may be 1,500 to 2,100 pg/mL, 1,600 to 2,000 pg/mL, or 1,700 to 1,900 pg/mL. When the dose is 36 mg/4 weeks, the maximum plasma concentration (Cₘₐₓ) may be 2,400 to 3,100 pg/mL, 2,500 to 3,000 pg/mL, 2,600 to 2,900 pg/mL, or 2,700 to 2,800 pg/mL. An oral formulation of finasteride showed a pattern in which the maximum plasma concentration was reached immediately after administration and then the blood concentration decreased rapidly, whereas in the case of the injectable composition of the present invention, there was a change in the value of the maximum plasma concentration depending on the dose, but the blood concentration gradually increased after administration, so that the maximum plasma concentration usually appeared between 500 and 700 hours, and after reaching the maximum plasma concentration, the blood concentration decreased steadily, so that the blood concentration of finasteride did not show a large difference between the highest and lowest levels.

The steady-state average concentration (C_{ss, avg}) of finasteride following administration of the injectable composition may be 1,000 to 6,000 pg/mL, 1,100 to 5,900 pg/mL, 1,200 to 5,800 pg/mL, 1,300 to 5,700 pg/mL, 1,400 to 5,600 pg/mL, 1,500 to 5,500 pg/mL, 1,600 to 5,400 pg/mL, or 1,700 to 5,300 pg/mL. The steady-state average concentration also varies depending on the dose. Specifically, when the dose is 12 mg/4 weeks, C_{ss,avg} may be 1,400 to 2,100 pg/mL, 1,500 to 2,000 pg/mL, 1,600 to 1,900 pg/mL, or 1,700 to 1,800 pg/mL, and when the dose is 24 mg/4 weeks, C_{ss,avg} may be 3,200 to 3,800 pg/mL, 3,300 to 3,700 pg/mL, or 3,400 to 3,600 pg/mL. When the dose is 36 mg/4 weeks, C_{ss,avg} may be 4,900 to 5,600 pg/mL, 5,000 to 5,500 pg/mL, 5,100 to 5,400 pg/mL, or 5,200 to 5,300 pg/mL. Through values within the above range, it was found that the injectable composition could exhibit excellent drug concentration in steady state.

AUC is determined by dose and clearance as shown in the following equation:
$Drug clearance = dose / AUC$

The clearance (CLs) is a model-independent parameter, but has a mathematical relationship with elimination rate constant (K) and distribution volume (V) as follows:
$CLs = V \times K$

As shown in this relationship equation, clearance and distribution volume determine the rate of drug elimination.

When clearance is constant, AUC is proportional to the dose, and thus when the drug dose is doubled, AUC increases by two times. According to this concept, it also means that clearance can be determined by AUC and dose.

Since the blood concentration according to the dose and time can be usually determined, AUC can be calculated, and thus the clearance can be calculated using the following equation:

Assuming a one-compartment model and administering a fixed dose of drug, AUC can be easily calculated as follows.$AUC = initial concentration \left(Co\right) / elimination rate constant \left(K\right)$

However, in the case of a drug in which the natural logarithm of the drug concentration versus time decreases linearly or nonlinearly, AUC can be calculated using a method called the "trapezoidal rule".

In the trapezoidal rule, the time versus blood concentration curve is considered as a series of connected trapezoids, and adjacent measured blood concentrations are connected by a straight line.

Although the upper part of the trapezoid is a curve, if the time interval (height of the trapezoid) is small, the curve may be assumed to be a straight line, and the resulting error is very small and neglectable. In this way, the area of each trapezoid is easily calculated, and the sum of the areas of all trapezoids becomes the total area under the concentration curve. When calculating AUC using the trapezoidal rule, blood concentrations are not replaced by natural logarithmic values. To calculate clearance, the AUC from time 0 to infinity should be used. The AUC from time 0 to infinity is also calculated using the trapezoidal rule, which uses the method of adding the calculated area from the last measured time to infinity to the AUC from time 0 to the time of the last concentration measurement. A large AUC value indicates relatively high bioavailability, which can be said to be an indicator of the amount absorbed, indicating how much is absorbed in the body.

The AUC at 0 hour to 672 hours (ACU₀₋₆₇₂ₕ) of finasteride following administration of the injectable composition of the present invention may be 500,000 to 2,000,000 hr*pg/mL, 510,000 to 1,900,000 hr*pg/mL, 511,000 to 1,800,000 hr*pg/mL, 512,000 to 1,700,000 hr*pg/mL, 513,000 to 1,600,000 hr*pg/mL, 514,000 to 1,600,000 hr*pg/mL, or 515,000 to 1,600,000 hr*pg/mL. The AUC also varies depending on the dose. Specifically, when the dose is 12 mg/4 weeks, the AUC may be 500,000 to 525,000 hr*pg/mL, 510,000 to 524,000 hr*pg/mL, 510,000 to 523,000 hr*pg/mL, 510,000 to 522,000 hr*pg/mL, 510,000 to 521,000 hr*pg/mL, or 510,000 to 520,000 hr*pg/mL, and when the dose is 24 mg/4 weeks, the AUC may be 1,000,000 to 1,300,000 hr*pg/mL, 1,010,000 to 1,200,000 hr*pg/mL, 1,020,000 to 1,100,000 hr*pg/mL, or 1,030,000 to 1,100,000 hr*pg/mL. When the dose is 36 mg/4 weeks, the AUC may be 1,100,000 to 1,800,000 hr*pg/mL, 1,200,000 to 1,700,000 hr*pg/mL, 1,300,000 to 1,600,000 hr*pg/mL, 1,400,000 to 1,600,000 hr*pg/mL, or 1,500,000 to 1,600,000 hr*pg/mL.

The steady-state AUC (AUCss) of finasteride following administration of the injectable composition may be 1,000,000 to 4,000,000 hr*pg/mL, 1,100,000 to 3,900,000 hr*pg/mL, 1,100,000 to 3,800,000 hr*pg/mL, 1,100,000 to 3,700,000 hr*pg/mL, or 1,100,000 to 3,600,000 hr*pg/mL. The steady-state AUC also varies depending on the dose. Specifically, when the dose is 12 mg/4 weeks, the steady-state AUC may be 1,000,000 to 1,300,000 hr*pg/mL, or 1,100,000 to 1,200,000 hr*pg/mL, and when the dose is 24 mg/4 weeks, the steady-state AUC may be 2,000,000 to 2,600,000 hr*pg/mL, 2,100,000 to 2,500,000 hr*pg/mL, 2,200,000 to 2,400,000 hr*pg/mL, or 2,300,000 to 2,400,000 hr*pg/mL. When the dose is 36 mg/4 weeks, the steady-state AUC may be 3,100,000 to 3,800,000 hr*pg/mL, 3,200,000 to 3,700,000 hr*pg/mL, 3,300,000 to 3,600,000 hr*pg/mL, or 3,400,000 to 3,600,000 hr*pg/mL. It was confirmed that excellent bioavailability was exhibited within the above range.

The injectable composition of the present invention comprises microparticles containing finasteride, wherein the microparticles contain finasteride and a biodegradable polymer, have the shape of a sphere uniformly containing finasteride and the biodegradable polymer, and have an average diameter of 30 to 50 µm.

The microparticles contain a biodegradable polymer and finasteride. In this case, the microparticles are not a capsule-type formulation, but are completely spherical microparticles produced from a composition comprising a uniform mixture of the biodegradable polymer and finasteride, and are characterized in that finasteride is uniformly distributed within the microparticles.

The average diameter of the microparticles is 30 to 50 µm, and the standard deviation of the average diameter is 3.0 to 5.5 µm.

The present invention relates to microparticles produced by the method for producing microparticles to be described later, wherein the microparticles are characterized in that a biodegradable polymer and finasteride are uniformly distributed therein as described above, the produced microparticles are characterized by having an average diameter of 30 to 50 µm, and the standard deviation of the average diameter is 3.0 to 5.5 µm.

That is, even though micro-sized particles are produced, the standard deviation of the average diameter is only 3.0 to 5.5 µm, indicating that particles of almost the same size may be produced.

The injectable composition comprising microparticles containing finasteride according to the present invention, when injected *in vivo*, may release finasteride in a sustained manner for at least 1 month, 1 month to 2 months, or 1 month to 3 months. More specifically, as described above, the microparticles of the present invention contain a biodegradable polymer, and as the biodegradable polymer is degraded *in vivo*, finasteride may be released *in vivo*.

The production of the microparticles containing finasteride according to the present invention is performed in the following order: step 1) (S100) of preparing a first mixture; step 2) (S200) of preparing a second mixture; step 3) (S300) of introducing the first mixture into a straight microchannel; step 4) (S400) of introducing the second mixture into a microchannel on both sides or one side; step 5) (S500) of collecting microparticles; step 6) (S600) of stirring the collected microparticles; and step 7) (S700) of washing and drying the microparticles.

More specifically, a method for producing the microparticles containing finasteride according to one embodiment of the present invention is described as follows.

Step 1) (S100) is a step of preparing a first mixture by dissolving a biodegradable polymer and finasteride in an organic solvent, wherein the biodegradable polymer is selected from the group consisting of polylactic acid, polylactide, polylactic-co-glycolic acid, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, polyamino acids, and combinations thereof mixtures thereof, with polylactide-co-glycolide (PLGA) being preferred, but the biodegradable polymer is not limited to the above examples.

In addition, the organic solvent is water-immiscible, and may be, for example, any one or more selected from the group consisting of chloroform, chloroethane, dichloroethane, dichloromethane, trichloroethane, and mixtures thereof, with dichloromethane being preferred, but the organic solvent is not limited to the above examples. In addition to the above-listed organic solvents, any organic solvent may be used without limitation, as long as it is capable of dissolving the biodegradable polymer and finasteride and may be easily selected by those skilled in the art.

Step 1) (S100) is a step of preparing the first mixture by dissolving the biodegradable polymer and finasteride, and as the solvent, the organic solvent described above is used. The organic solvent is used to completely dissolve finasteride and the biodegradable polymer based on the dissolution properties thereof. After complete dissolution, the first mixture contains the biodegradable polymer and finasteride at a weight ratio of 2:1 to 15:1.

If the weight ratio between the biodegradable polymer and finasteride is less than 2:1, that is, if the content of the biodegradable polymer is lower than the lower limit of the above weight ratio, a problem may arise in that, because the content of the biodegradable polymer is lower than the content of finasteride, it is difficult to produce microparticles in which finasteride is uniformly distributed in spherical biodegradable polymer particles. If the weight ratio between the biodegradable polymer and finasteride is more than 15:1, that is, if the content of the biodegradable polymer is higher than the upper limit of the above weight ratio, a problem may arise in that, because the content of finasteride in the microparticles is low, a large amount of the microparticles need to be administered in order to administer the drug at a desired concentration.

More specifically, the content of the biodegradable polymer in the first mixture is 10 to 20 wt%, preferably 12.5 to 15 wt%, without being limited thereto.

Step 2) (S200) is a step of preparing a second mixture by dissolving a surfactant in water. As the surfactant, any surfactant may be used without limitation as long as it may help the biodegradable polymer solution to form a stable emulsion. Specifically, the surfactant may be any one or more selected from the group consisting of nonionic surfactants, anionic surfactants, cationic surfactants, and mixtures thereof, and more specifically, may be any one or more selected from the group consisting of methylcellulose, polyvinylpyrrolidone, lecithin, gelatin, polyvinyl alcohol, polyoxyethylene sorbitan fatty acid ester, polyoxyethylene castor oil derivatives, sodium lauryl sulfate, sodium stearate, ester amine, linear diamine, fatty amines, and mixtures thereof, with polyvinyl alcohol being preferred, but the surfactant is not limited to the above examples.

Step 3) (S300) and step 4) (S400) are steps of introducing the first mixture and the second mixture into microchannels formed on a wafer and allowing the first mixture and the second mixture to flow therethrough.

More specifically, the microchannels may be formed on a material selected from the group consisting of a silicon wafer and a polymer film, but the material is not limited to the above examples, and it is possible to use any material on which the microchannels may be formed.

The polymer film may be selected from the group consisting of polyimide, polyethylene, fluorinated ethylene propylene, polypropylene, polyethylene terephthalate, polyethylene naphthalate, polysulfone, and mixtures thereof, without being limited thereto.

As an example, aluminum is deposited on a silicon wafer using an e-beam evaporator, and photoresist is patterned on the aluminum using a photolithography technique. Thereafter, the aluminum is etched using the photoresist as a mask, the photoresist is removed, the silicon wafer is etched by deep ion reactive etching (DRIE) using the aluminum as a mask, the aluminum is removed, and then glass is anodically bonded onto the wafer and hermetically sealed, thereby fabricating the microchannels.

The microchannel may have a horizontal width of 100 µm and a vertical depth of 80 µm, and in order to control the variation in the injection amount (speed) of the first mixture, a resistance channel may be positioned at the front of the microchannel so that the first mixture may pass through the resistance channel and may be introduced into the microchannel at a constant injection amount (speed). The microchannel may have a horizontal width of 20 µm and a vertical depth of 100 µm. The microparticles have an average diameter of 30 to 50 µm, preferably 40 µm, without being limited thereto. If the average diameter of the microchannel is 35 µm or less, small-sized microparticles with a diameter of 20 µm or less may be produced, which are more likely to be phagocytosed by macrophages after injection into the human body, which may affect the release and *in vivo* absorption of the effective drug. In addition, if the produced microparticles have an average size of more than 55 µm, foreign body sensation and pain may increase when the microparticles are administered by injection, and the particle size distribution of the produced microparticles may increase, making it difficult to produce microparticles having a uniform particle size.

However, the average diameter of the microchannel may be changed depending on the range of the pressure at which the first or second mixture is introduced. For example, when the diameter of the microchannel is 100 µm, the second mixture should be introduced at a pressure of 1,000 to 2,000 mbar, and the first mixture may be introduced at a pressure of 500 to 1,000 mbar.

The average diameter of the microchannel is closely related not only to the average diameter of the microparticles, but also to the pressure of introduction of the first mixture and the second mixture. Thus, the average diameter of the microchannel is not limited to the above-described example and may be changed depending on the average diameter of the microparticles to be produced or the pressure of introduction of the first mixture and the second mixture.

In addition, the cross-sectional width (w) and cross-sectional height (d) of the microchannel are closely related to the average diameter (d') of the microparticles to be produced. The cross-sectional width (w) of the microchannel is in the ratio range of 0.3 to 0.5 with respect to the average diameter (d') of the microparticles, and the cross-sectional height (d) of the microchannel is in the ratio range of 0.3 to 0.8 with respect to the average diameter (d') of the microparticles.

Step 3) (S300) is a step of introducing the first mixture into a straight microchannel and allowing the first mixture therethrough, and step 4) (S400) is a step of introducing the second mixture into a microchannel formed on both sides or one side to form a junction with the straight microchannel and allowing the second mixture to flow therethrough.

In other words, the first mixture flows along the straight microchannel, and the second mixture flows along a microchannel, formed on both sides or one side of the straight microchannel and forming a junction with the straight microchannel, and meets the flow of the first mixture.

When the first mixture is introduced into the straight microchannel, it is introduced under a constant pressure condition and allowed to flow at a constant flow rate. Here, the pressure condition is 500 to 1,500 mbar, preferably 750 to 1,100 mbar, without being limited thereto.

In addition, when the second mixture is introduced into the microchannel on both sides or one side, it is introduced under a constant pressure condition and allowed to flow at a constant flow rate. Here, the pressure condition is 1,000 to 2,500 mbar, preferably 1,400 to 2,300 bar, without being limited thereto.

That is, in order to make the second mixture intersecting with the flow of the first mixture flow at a higher flow rate than the first mixture to be introduced into the straight microchannel, the second mixture is allowed to flow under a higher pressure condition.

By making the flow rates of the first mixture and the second mixture different and making the flow rate of the second mixture higher than the flow rate of the first mixture as described above, the second mixture with a relatively higher flow rate compresses the first mixture at the point where the flow of the second mixture meets the flow of the first mixture, and at this time, the biodegradable polymer and finasteride in the first mixture produce spherical microparticles due to the repulsive force between the first mixture and the second mixture. More specifically, the formed microparticles have a structure in which finasteride is evenly distributed in the spherical biodegradable polymer.

Step 5) (S500) is a step of collecting the microparticles. In this step, the microparticles are collected in a bath containing the second mixture to prevent aggregation of the initially produced microparticles.

Step 5) (S500) is performed using the second mixture prepared in step 2) (S200), that is, a mixed solution of the surfactant and water. Specifically, a portion of the second mixture prepared in step 2) (S200) is introduced into the microchannel, and the other portion is transferred into the bath in step 5) (S500) and used to prevent aggregation of the collected microparticles.

Step 6) (S600) is a step of stirring the collected microparticles in the bath. In this step, the microparticles are stirred at a predetermined stirring speed at a predetermined temperature, thereby evaporating and removing the organic solvent present on the surfaces of the microparticles. Specifically, step 6) comprises the sequential steps of: subjecting the microparticles to first stirring at a speed of 100 to 500 rpm at 15 to 20°C for 0.5 to 3 hours while raising the temperature to 35 to 45°C; subjecting the microparticles to second stirring at a speed of 100 to 500 rpm at 35 to 45°C for 4.0 to 24 hours; and subjecting the microparticles to third stirring at a speed of 100 to 500 rpm for 0.5 to 1.5 hours while cooling to 15 to 25°C.

Step 6) is characterized in that the stirring temperature is higher in the second stirring step than in the first stirring step. By raising the temperature stepwise, it is possible to control the evaporation rate of the organic solvent present on the surfaces of the microparticles. That is, it is possible to slowly evaporate the organic solvent present on the surfaces of the microparticles, thereby producing microparticles having a smooth surface.

The temperature at which the first mixture and the second mixture flow through the microchannels is also 15 to 20°C, preferably 17°C. That is, after the mixtures flow along the microchannels and intersect each other to produce microparticles, the collected microparticles are maintained at a constant low temperature of 15 to 20°C until they are stirred in the first stirring step. It is possible to produce and maintain spherical particles only when the microparticle production process is maintained at a low temperature. That is, if a lowtemperature condition is not used, a problem arises in that it is difficult to produce particles having a uniform spherical shape.

Lastly, step 7) (S700) is a step of washing and drying the microparticles. In this step, the microparticles from which the organic solvent on the surfaces has been completely removed by stirring are washed several times with sterile filtered purified water to remove the surfactant remaining on the microparticles, and then the microparticles are vacuum-dried or freeze-dried.

The microparticles finally produced have a structure in which the finasteride drug is uniformly distributed in the spherical biodegradable polymer microparticles, and contain the biodegradable polymer and finasteride at a weight ratio of 2:1 to 15:1.

The weight ratio between the biodegradable polymer and finasteride contained in the microparticles is the same as the weight ratio in the first mixture. Specifically, as the microparticles are produced and the organic solvent is completely evaporated and removed therefrom, the produced microparticles may contain the biodegradable polymer and finasteride at the same weight ratio as the weight ratio in the first mixture.

A novel long-acting injectable composition for preventing or treating androgenetic alopecia according to another embodiment of the present invention may comprise: microparticles containing finasteride; and a suspending solvent.

The injectable composition is in a form wherein the microparticles are uniformly contained in the suspending solvent. When the injectable composition is administered, the microparticles themselves may be injected into the body, thereby exhibiting the effect of administering finasteride for a long period of time.

More specifically, when the microparticles are injected into the body, the effect of releasing finasteride by degradation of the biodegradable polymer may be exhibited, and at this time, since the microparticles of the present invention are in a form where the biodegradable polymer and finasteride are uniformly mixed together, they may exhibit the effect of administering a constant concentration of finasteride for a long period of time.

That is, when the injectable composition of the present invention is injected once, finasteride may be released in a sustained manner *in vivo* for at least 1 month, 1 to 2 months, or 1 to 3 months, and thus the problem of having to take the drug every day can be solved, thereby increasing user convenience.

The suspending solvent contains an isotonic agent, a suspending agent, and a solvent.

More specifically, the isotonic agent may be selected from the group consisting of D-mannitol, maltitol, sorbitol, lactitol, xylitol, sodium chloride, and mixtures thereof, with D-mannitol being preferred, but the isotonic agent is not limited to the above examples.

The suspending agent is selected from the group consisting of sodium carboxymethylcellulose, polysorbate 80, starch, starch derivatives, polyhydric alcohols, chitosan, chitosan derivatives, cellulose, cellulose derivatives, collagen, gelatin, hyaluronic acid (HA), alginic acid, algin, pectin, carrageenan, chondroitin, chondroitin sulfate, dextran, dextran sulfate, polylysine, titin, fibrin, agarose, fluran, xanthan gum, and mixtures thereof, with sodium carboxymethylcellulose and polysorbate 80 being preferred, but the suspending agent is not limited to the above examples.

As the solvent, water for injection may be used, and any solvent that may be used as water for injection may be used without limitation.

### Production of Microparticles Containing Finasteride

### 1. Production of Sustained-Release Particles (Main Drug) Containing Finasteride

(1) A first mixture was prepared by dissolving polylactide-co-glycolide (PLGA) and finasteride in dichloromethane and then subjected to sterile filtration.

Here, the content of polylactide-co-glycolide in the first mixture was 12.5 wt%, and the weight ratio between polylactide-co-glycolide and finasteride was 2:1.
(2) A second mixture containing 0.25 wt% of polyvinyl alcohol was prepared by mixing polyvinyl alcohol as a surfactant with water and then subjected to sterile filtration.
(3) The first mixture and the second mixture were introduced into microchannels formed on a silicon wafer and allowed to flow. Here, in order to allow each of the first mixture and the second mixture to flow at a constant flow rate, the first mixture was allowed to flow under a pressure condition of 750 mbar, and the second mixture was allowed to flow under a pressure condition of 1,400 mbar. The temperature condition was maintained at 17°C.
(4) Sustained-release muicroparticles produced at the intersection between the flow of the first mixture and the flow of the second mixture were collected in a bath containing the second mixture. The sustained-release microparticles collected in the bath were stirred at a speed of 150 rpm for 2 hours while raising the temperature in the bath to 42°C, and then stirred at a speed of 150 rpm for 10 hours while maintaining the temperature at 42°C.
(5) The temperature was then adjusted to 17°C.
(6) After completion of stirring, the sustained-release microparticles were washed several times with sterile filtered purified water, and the remaining water was removed. Then, the sustained-release microparticles were dried for 24 hours while maintaining the vacuum inside the bath at 50 mbar or less.

### Preparation of Composition for Subcutaneous Injection

(1) The sustained-release microparticles (main drug) produced in 1 were added to 2.0 mL (based on one vial) of a suspending solvent and then uniformly suspended, thereby preparing a composition for subcutaneous injection.
(2) The suspending solvent had the following composition.
   - Volume: 2.0 mL
   - Isotonic agent D-Mannitol: 100.0 mg
   - Suspending agent sodium carboxymethylcellulose: 10.0 mg
   - Suspending agent Polysorbate 80: 2.0 mg
   - Water for injection as solvent: remainder

### Study Method

### Subjects

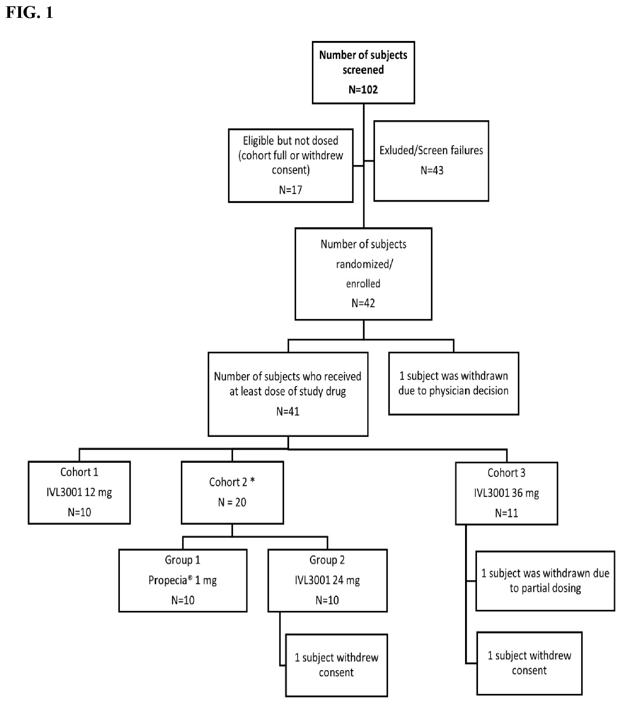

The study in the present invention was conducted in accordance with Therapeutic Goods Administration and Human Research Ethics Committee guidelines. The study was conducted at the Phase 1 Unit of Nucleus Network Pty Ltd in Brisbane, Australia from October 2021 to March 2022. The entire study was conducted in accordance with the provisions of the Declaration of Helsinki and Good Clinical Practice guidelines, and the protocol was registered at www.clinicaltrials.gov (NCT04945226; registration date: June 30, 2021). Healthy men aged 18 to 55 years with a body mass index (BMI) of 18 to 32 kg/m² participated in this study. Potential subjects completed a signed informed consent form and were subjected to screening prior to the study. A total of 40 healthy, non-smoking adult men were included in the study across three cohorts (FIG. 1).

This study was approved by the Alfred Hospital Ethics Committee. The ethics committee reference number is Project 364/20. Informed consent was obtained from all study subjects.

All subjects underwent regular check-ups, which included measurement of vital signs (blood pressure, heart rate, and body temperature), 12-lead electrocardiogram (ECG), serological tests (hepatitis B surface antigen, hepatitis C virus antibodies, and HIV antigen/antibody), health status, and routine laboratory tests (CBC, biochemistry tests, and urine analysis).

The main inclusion criteria are as follows:
(1) Healthy males aged 18 to 55 years or older at the time of screening test, who are non-smokers or moderate or light smokers (less than 10 cigarettes per day or nicotine equivalent) and agree to quit smoking from 48 hours before first IP administration;
(2) subjects who meet screening test criteria and are considered to be in excellent health by the investigator;
(3) subjects with a BMI between ≥ 18 kg/m² and ≤ 32 kg/m² and a minimum body weight between ≥ 50 kg and ≤ 100 kg at the time of screening test;
(4) ability and willingness to report to and stay in the clinical research unit overnight when necessary; and
(5) subjects who voluntarily participated in the study, received and fully understood a detailed explanation of the study, and agreed in writing to comply with the instructions.

The main exclusion criteria are as follows:
(1) prior or ongoing medical conditions, medical history, physical examination findings, or screening test abnormalities that, in the opinion of the investigator, could adversely affect the safety of the subjects;
(2) presence or history of any clinically significant blood, kidney, endocrine, lung, gastrointestinal tract, cardiovascular, liver, or neurological condition;
(3) subjects with mild depression and anxiety;
(4) subjects with known allergy or hypersensitivity to finasteride or a history of drug abuse;
(5) Age-adjusted prostate-specific pathogen at screening: 0 to 2.5 ng/mL for subjects under 50 years of age, 0 to 4 ng/mL for subjects over 50 years of age (unless the investigator determines that it is not clinically important);
(6) Individuals who participated in another human study within 2 months before the start of the study;
(7) Alcohol abuse or dependence within the past 3 months;
(8) Hypersensitivity to the drug finasteride or any excipient of IPs; however, past use of finasteride was permitted; and
(9) People who were deemed ineligible for participation in this clinical study for other reasons defined by the investigator.

### Study Design

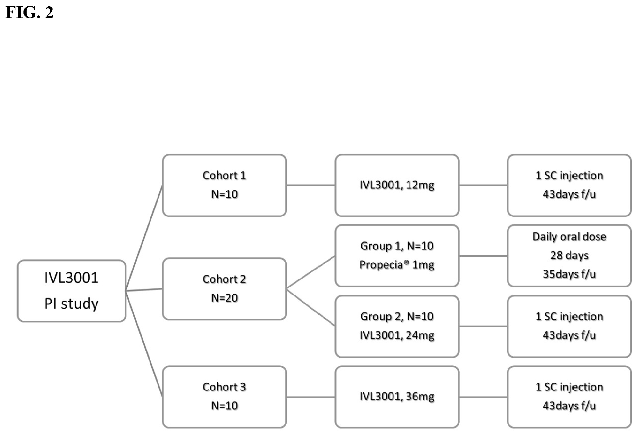

A total of 40 non-smoking, healthy adult males were recruited for this study. Subjects who passed the screening process and were deemed eligible for the study were assigned to three groups. Cohort 1 received a single SC injection of 12 mg IVL3001, and cohort 3 received a single SC injection of 36 mg IVL3001. Each subject was followed up until day 43. In contrast, cohort 2 was divided into two groups. Group 1 received 1 mg finasteride (Propecia^{®}) orally once daily for 28 days and was followed up until day 35, and group 2 received a single subcutaneous injection of 24 mg IVL3001 and was followed up until day 43. On the morning of day 1, each subject in the three cohorts received a single subcutaneous injection of IVL3001 or received daily oral finasteride, and was discharged from the hospital on day 3. The subjects returned to the hospital according to a pre-determined schedule for 43 or 35 days and were assessed for tolerability, PK, and PD. In this study, once-monthly administration of the sustained-release formulation IVL3001 was compared with daily oral administration of finasteride. In this study, the plasma drug concentration was analyzed on day 28 to investigate the comparative effect. FIG. 2 shows a schematic diagram showing the study design.

Cohort 1 received a single SC injection of 12 mg IVL3001 and was followed up (f/u) until day 43. Cohort 2 was divided into two groups. Group 1 of cohort 2 received 1 mg finasteride orally once daily for 28 days and was followed up (f/u) until day 35, and group 2 of cohort 2 received a single SC injection of 24 mg IVL3001 and was followed up (f/u) until day 43. Similarly, cohort 3 received a single SC injection of 36 mg IVL3001 and was followed up (f/u) until day 43.

### Measurement of Plasma Concentrations of Finasteride, DHT and Testosterone

Plasma finasteride, DHT, and testosterone concentrations were measured using liquid chromatography-tandem mass spectrometry (LC-MS). Calibration standards and test samples for testosterone and DHT LC-MS/MS were prepared using liquid/liquid extraction. Testosterone and DHT analyses were performed using LC-MS/MS (Sciex, USA). After plasma extraction, the analytes were eluted through an ACE Excel 2 C18 column using a gradient profile of solvent at a flow rate of 0.5 mL/min. After optimization using electrospray ionization in positive mode, each precursor/product ion pair for finasteride, testosterone, and DHT were used for analysis. Finally, the samples containing steroids were recovered. The lower limit of quantitation for testosterone D5 was 100 pg/mL, and 50 pg/mL was used as the internal standard for DHT measurement.

### Endpoints

### Primary Endpoint

The primary endpoint of this study is to determine safety. This was assessed by measuring vital signs, 12-lead ECG, serological tests, and other blood and biochemical tests. Injection site reactions were assessed at 0.5, 1, 12, and 48 hours using a global severity assessment. Severity was assessed on a 4-point scale, with scores ranging from 0 to 3, where 0 represents no response, 1 represents mild pain, 2 represents pain distribution, and 3 represents unbearable pain. Injection site reactions were assessed at every outpatient visit after discharge. In addition, PK parameters were derived using a non-compartmental analysis approach based on venous blood samples obtained on days 1, 2, 3, 5, 8, 10, 15, 22, 29, 35, and 43 in the IVL3001 group and on days 2, 3, 8, 15, 22, 28, 29, 30, and 35 in the finasteride group. Whole blood was collected and centrifuged to obtain plasma. Plasma finasteride concentrations were measured using a validated LC-MS method. Maximum plasma concentration (Cmax), time to reach Cmax (Tmax), areas under the curve for 24 and 672 hours after dosing (AUC₀₋₂₄, and AUC₀₋₆₇₂), areas under the curve from time 0 to the last quantified concentration and to infinity (AUC₀₋ₜ, and AUC_{0-inf}), total plasma clearance, apparent total volume of distribution based on steady state or terminal phase, and elimination half-life were assessed.

### Secondary Endpoints

DHT and testosterone measurements are secondary endpoints. DHT and testosterone were measured as pharmacodynamic markers and were assessed at the same time points as the PK sampling times. Additionally, in this study, changes in DHT concentrations with testosterone concentrations during and at the end of treatment in subjects who received IVL3001 or finasteride 1 mg tablets were evaluated and compared. Plasma DHT and testosterone concentrations were measured using a validated LC-MS method. All biochemical and chromatographic analyses, including LC-MS, were performed by Syneos Health Clinique Inc. (Quebec, Canada) and Sydpath Clinical Trials (NSW, Australia).

### Modeling and Simulation

PK and PD modeling were performed to determine the therapeutic dose in AGA. A two-compartment PK model using observed clinical study data was adopted. To predict and compare the efficacy of the test drug (IVL3001) and the reference drug (finasteride 1 mg tablet), the Emax model was directly used for individual plasma DHT concentrations to predict the efficacy of finasteride in both the test and reference drugs, which was then incorporated into the PK model. The effective dose for additional clinical studies was estimated using established PK/PD models. All PK/PD modeling and simulations were performed using Phoenix WinNonlin, v.8.3. DHT and testosterone levels (uncorrected and % baseline corrected) were listed and summarized by nominal sampling time and treatment. Mean ± SD profiles of individual subjects and PD data were plotted by treatment using actual and nominal times, respectively.

### Safety Measurement

The clinical status and vital signs of the subjects were evaluated before and after participation in the study to investigate adverse effects. Hematological parameters such as white blood cell, red blood cell, hemoglobin, hematocrit, and platelet counts were analyzed. Adverse effects (AEs) were recorded in terms of symptoms and signs, duration, intensity, relationship to study drug, action taken, outcome, and seriousness. All biochemical analyses were performed in the clinical laboratory at Mater Hospital, Brisbane, Australia.

### Statistical Analysis

Statistical analysis was performed using SAS v.9.4 (SAS Institute, Cary, NC, USA). All information related to the safety of the subjects was summarized using descriptive statistics. AEs were also classified using the Medical Dictionary for Regulatory Activities (MedDRA) version 24.0 (MedDRA MSSO, McLean, VA, USA) and summarized by system organ class, preferred term, severity, and relationship to IVL3001 or finasteride. PK analysis was based on a non-compartmental analysis approach and was performed in the PK population (patients who received at least one dose and had evaluable PK data). PK parameters were calculated using plasma concentrations and actual blood sampling times using Phoenix WinNonlin v.8.3 (Certara, Princeton, NJ, USA) and summarized.

### Results

### Demographic Characteristics of Subjects

A total of 42 male subjects were enrolled, and 41 subjects met the criteria to receive the study drug. Of these, 38 subjects completed the study, 1 subject did not receive the full dose, and 2 subjects withdrew consent due to reasons unrelated to adverse effects. Most of the subjects were of European descent (93.3%), and the mean age of the subjects was 32.4±9.6 years. The process for screening the study subjects is shown in FIG. 2. The general demographic characteristics of the subjects are shown in Table 1 below. There were no statistically significant differences in age, height, weight, or BMI.

**[Table 1]**

| | IVL3001 12mg SC (N=11) | IVL3001 24mg SC (N=10) | IVL3001 36mg SC (N=11) | Finasteride 1mg (QD) (N=10) | Overall (N=42) |
|---|---|---|---|---|---|
| Age, mean(SD), y | 33.5 (9.1) | 33.7 (13.0) | 29.2 (9.4) | 33.4 (6.8) | 32.4 (9.6) |
| Sex, n (%) Male | 11 (100) | 10 (100) | 11 (100) | 10 (100) | 42 (100) |
| Race, n (%) | | | | | |
| European descent | 6 (54.5) | 10 (100) | 7 (63.6) | 7 (70.0) | 30 (71.4) |
| African-American | 1 (9.1) | 0 | 1 (9.1) | 0 | 2 (4.8) |
| S-E Asian | 4 (36.4) | 0 | 2 (18.2) | 1 (10.0) | 7 (16.7) |
| Other | 0 | 0 | 1 (9.1) | 2(20.0) | 3(10.0) |
| Height, mean (SD), cm | 179.5 (7.6) | 180.0 (6.3) | 177.6 (9.2) | 176.9 (4.7) | 178.5 (7.0) |
| Weight, mean (SD), kg | 79.7 (10.8) | 77.8 (9.3) | 77.8 (8.8) | 82.0 (13.2) | 79.3 (10.4) |
| BMI, mean(SD) kg/m² | 24.8 (2.9) | 24.3 (3.5) | 24.8(2.7) | 26.3 (4.7) | 25.0 (3.4) |

### Safety

Forty treatment-emergent adverse events (TEAEs) were observed in 23 of 41 patients (56.1%) who received IVL3001 or finasteride. Thirty-seven TEAEs were reported in 20 of 31 subjects (64.5%) who received a single SC dose of IVL3001, whereas three TEAEs were reported in three of 10 subjects (30.0%) who received finasteride 1 mg tablets. A dosedependent trend was observed in the IVL3001 treatment groups with respect to the number of TEAEs reported. However, the number and percentage of patients reporting TEAEs were similar across the IVL3001 treatment groups. Overall, the majority of reported TEAEs (18, 43.9%) were considered study drug-related and resolved without medical intervention by the end of the study. Most injection site reactions occurred in the study drug group, and the incidence rates were similar to those in the finasteride group, except for injection site reactions. Most injection site reactions were mild, and the incidence rates were similar across dose groups (Table 2). No safety concerns were observed. Vital signs and biochemical parameters (hematology, blood chemistry, ECG, physical examination, and urinalysis) were determined to be within reference ranges and were not of clinical significance.

**[Table 2]**

| | IVL3001 12mg (SC) (n=10) | IVL3001 24mg (SC) (n=10) | IVL3001 36mg (SC) (n=11) | Finasteride 1mg (QD) (n=10) | Overall (n=41) |
|---|---|---|---|---|---|
| | Participants, n (%) | | | | |
| AEs by MedDRA system organ class MedDRA preferred items | | | | | |
| Subjects with at least one TEAE | 6 (60.0) | 7 (70.0) | 7 (63.6) | 3 (30.0) | 23 (56.1) |
| General disorders and administration site condition | 6 (60.0) | 4 (40.0) | 6 (54.5) | 0 | 16 (39.0) |
| Injection site bruising | 6 (60.0) | 0 | 0 | 0 | 6 (14.6) |
| Injection site induration | 0 | 2 (20.0) | 4(36.4) | 0 | 6 (14.6) |
| Injection site pain | 0 | 1 (10.0) | 4(36.4) | 0 | 5 (12.2) |
| Injection site Haematoma | 0 | 2 (20.0) | 1(9.1) | | 3 (7.3) |
| Nervous system disorder | 1(10.0) | 1 (10.0) | 0 | 3 (30.0) | 5 (12.2) |
| Headache | 0 | 1 (10.0) | 0 | 3 (30.0) | 4 (9.8) |
| Infections and infestations | 0 | 0 | 3 (27.3) | 0 | 3 (7.3) |
| COVID-19 | 0 | 0 | 3 (27.3) | 0 | 3 (7.3) |
| Investigations | 1 (10.0) | 2 (20.0) | 0 | 0 | 3 (7.3) |
| Blood creatine phosphokinase increased | 0 | 2 (20.0) | 0 | 0 | 2 (4.9) |

### Pharmacokinetic Analysis

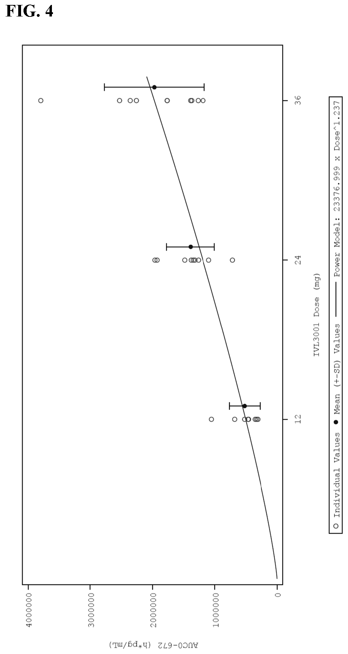

Plasma concentration profiles following administration of IVL3001 doses (12, 24, and 36 mg) showed a prolonged absorption phase, and reached peak levels between 500 and 700 hours after administration, in contrast to a sharp peak and decline following oral administration of finasteride 1 mg once daily (QD). The main PK difference observed over the 672 hours following SC administration of IVL3001 was that finasteride plasma concentrations were more consistent and had fewer abrupt peak and trough fluctuations, and abrupt peak and trough fluctuations are typical in daily oral administration of finasteride (FIG. 4).

The mean elimination half-life (t_{1/2el}) of finasteride was 6.4 hours (day 1) and 6.9 hours (day 28). After IVL3001 administration, the t_{1/2el} was between 100 and 160 hours, and the CL/F of IVL3001 and the oral tablet were similar. The PK of IVL3001 administered at the highest dose (36 mg) during the first 24 hours was similar to that of the reference oral formulation. However, the PK of 24 mg IVL3001 during the 28-day exposure was similar to that of oral finasteride 1 mg tablets, with a geometric mean ratio (IVL3001 24 mg/finasteride 1 mg QD) of 1.406 (90% CI: (1.003, 1.973). AUC₀₋₂₄ and Cₘₐₓ were lower in all IVL3001 groups than in the oral finasteride 1 mg tablet group. Therefore, it was confirmed that there was no initial burst when the IV13001 formulation was administered. The AUC₀₋₆₇₂, AUC_{0-inf}, and Cₘₐₓ values of IVL3001 met the proportionality criteria (FIG. 4).

### Pharmacodynamic Analysis

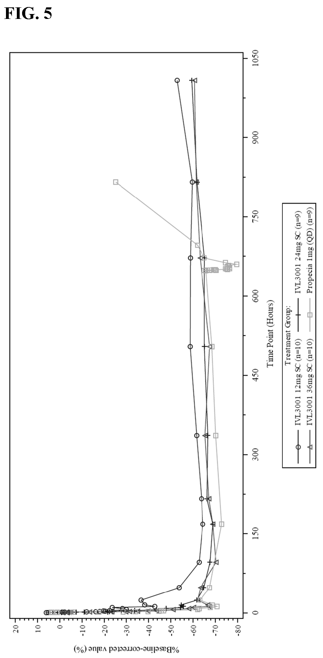

The baseline-corrected median DHT values (%) at all time points ranged from - 64.35 to 5.99%, -68.93 to 0.96%, and -70.36 to -1.25% for at 12 mg, 24 mg, and 36 mg doses of IVL3001, respectively. Similarly, the baseline-corrected median DHT values (%) at all time points ranged from -74.04 to 3.58% for oral finasteride 1 mg tablets. An inhibitory effect on DHT was observed at all concentrations of IVL3001 and oral finasteride 1 mg tablets, with baseline-corrected median testosterone levels initially decreasing and then increasing significantly over time (FIG. 5).

### Simulation

A two-compartment PK model for IVL3001 and oral finasteride 1 mg tablets was successfully developed using plasma finasteride concentration-time profiles obtained from a clinical study of single doses of IVL3001 (12, 24, and 36 mg) and repeated doses of oral finasteride 1 mg tablets (1 mg QD for 28 days).

Simulation results showed that IVL3001 had a very low absorption rate constant and clearance profile compared to oral finasteride 1 mg tablets, and had a higher distribution profile in the central compartment (Table 3).

**[Table 3]**

| Parameters | Estimates | |
|---|---|---|
| | Finasteride (1 mg) | IVL3001 |
| tvKa (h⁻¹) | 0.485 | 0.001 |
| tvV (L) | 81.6 | 1000.9 |
| tvV2 (L) | 373.2 | 0.217 |
| tvCL (L/h) | 18.4 | 10.0 |
| tvCL2 (L/h) | 18.9 | 0.01 |

wherein
tvKa: first-order absorption rate constant; tvV: distribution volume (central compartment); tvV2: distribution volume (peripheral compartment); tvCL: clearance from central compartment, tvCL2: clearance between central compartment and peripheral compartment; finasteride 1 mg: (Propecia^{®}).

The PK profiles were simulated using the established PK model after repeated administration of IVL3001 or oral finasteride 1 mg tablets. After administration of IVL3001 12, 24, and 36 mg every 4 weeks, the PK profiles of oral finasteride 1 mg tablets were within the concentration range between the C_{ss, min} and C_{ss, max} of the oral finasteride 1 mg tablet group (Table 4).

**[Table 4]**

| Parameters | Finasteride (1 mg QD) | IVL3001 | | |
|---|---|---|---|---|
| | | 12 mg/Q4W | 24 mg/Q4W | 36 mg/Q4W |
| Cₘₐₓ (pg/mL) | 4,672.9 | 928.8 | 1,857.5 | 2,786 |
| C_{ss,avg} (pg/mL) | 2,244.5 | 1,751.6 | 3,503.3 | 5,252.7 |
| AUC₀₋₆₇₂ₕ (hr*pg/mL) | 1,463,862 | 519,096.5 | 1,038,193 | 1,557,070.1 |
| AUCₛₛ (hr*pg/mL) | 1,508,321 | 1,177,098 | 2,354,195 | 3,529,788.7 |

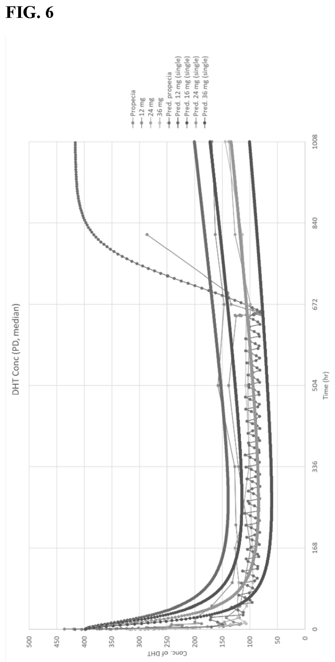

These study results suggest that IVL3001 has no safety concerns after administration at doses of 12, 24, and 36 mg. Plasma DHT concentrations determined in the clinical study were used to develop the PD model. The direct Emax model with ke (the elimination rate constant from the effect compartment), EC₅₀ (the half-maximal effective concentration), and E0 (the reference level) was used as the core structure of the PD model for DHT. Based on the DHT concentration profile, it was predicted that administering 12 to 36 mg of IVL3001 every 4 weeks would achieve plasma concentrations similar to oral finasteride 1 mg tablets, with comparable DHT reduction (FIG. 6).

A major obstacle to hair loss treatment in AGA patients is poor adherence to prescribed oral treatment. A major problem in AGA treatment is partial non-compliance, which can result in loss of benefits gained from oral medication over the years. This problem has been partly alleviated by the increasing use of long-acting injectables (LAIs), particularly in the management of patients who tend to be noncompliant with treatment. However, the recent development of second-generation LAIs has led clinicians to consider their use, particularly in the treatment of schizophrenia at the early stages of the disease.

LAIs have several advantages over oral administration. LAIs can alleviate withdrawal symptoms associated with non-compliance and maximize the range of pharmacodynamic effects. Likewise, first-pass metabolism does not affect LAIs, which can reduce the possibility of drug-drug interactions.

In this study, the highest dose (36 mg IVL3001) SC formulation was very similar to the oral formulation on a 24-hour basis, but over the 28-day exposure period, 24 mg IVL3001 was most similar to Propecia 1 mg tablets administered daily for 28 days. This result is particularly relevant in cases where patients are non-compliant with their prescribed oral medications, implying that LAIs may have an important role in the treatment of AGA.

Next, the importance of PK/PD analysis can be understood from the correlation between drug concentration (PK) and pharmacological effect (PD). As observed in the study, a single dose of IVL3001 achieved and maintained effective plasma concentrations for more than 28 days without an initial burst effect. The sustained and rapid plasma concentrations observed within 30 minutes after administration suggest that IVL3001 may offer more convenient and longer-lasting therapeutic effects than formulations that are taken orally daily.

Specifically, administration of IVL3001 of the present invention resulted in peak concentrations observed between 500 and 700 hours after administration, in contrast to the rapid rise and fall observed following oral administration of oral Propecia 1 mg QD. Here, SC IVL3001 finasteride plasma concentrations appeared to remain consistent up to 672 hours, whereas typical trough levels were observed when finasteride was orally administered daily.

As expected, for IVL3001 of the present invention, Cmax measured at various doses was consistent with the selected concentration. In addition, C_{ss,avg} observation results showed excellent plasma drug concentration at steady state. In addition, pharmacodynamic analysis results indicated that IVL3001 exhibited significantly better initial absorption rate, distribution volume, and reduced clearance parameters compared to finasteride. Furthermore, pharmacodynamic analysis results showed that the efficacy of IVL3001 was similar to that of finasteride 1 mg QD when administered at doses higher than 24 mg Q4W, demonstrating the efficacy of IVL3001 SC injection. In addition, the half-life (t½) of IVL3001 longer after SC injection was longer than that of finasteride, which is consistent with previous reports of similar studies conducted using other drugs. The remarkable bioavailability and bloodbrain barrier permeability of finasteride by SC injection of IVL3001 may explain these excellent results.

Therefore, despite similar clearance (CL/F), there is a significant difference in the elimination half-life (t_{1/2el}) when the same drug is administered via different routes, and in this study, the mean elimination half-life of oral finasteride was 6.4 hours on day 1 and 6.9 hours on day 28. However, after IVL3001 administration, the t_{1/2el} ranged from 100 to 160 hours. Extensive hepatic metabolism converts finasteride primarily to inactive metabolites, which can then be eliminated via the biliary and urinary routes. Subcutaneous administration bypasses the hepatic first-pass effect, allowing a greater proportion of the administered dose to reach systemic circulation. This may be due to the longer elimination half-life because the drug is exposed to systemic circulation without undergoing significant hepatic metabolism, as is the case with orally administered finasteride. This means that the increased stability of subcutaneously administered IVL3001 may exhibit the effect of releasing the drug in a sustained and controlled manner, potentially resulting in a more predictable and uniform therapeutic effect than the oral formulation.

Although the preferred embodiments of the present invention have been described in detail above, the scope of the present invention is not limited thereto, and various modifications and improvements made by those skilled in the art using the basic concept of the present invention as defined in the appended claims also fall within the scope of the present invention.

### Industrial Applicability

The present invention relates to a novel long-acting injectable composition for preventing or treating androgenetic alopecia containing finasteride.

## Claims

1. A novel long-acting injectable composition for preventing or treating androgenetic alopecia, which contains finasteride at a dose of 10 mg to 40 mg and inhibits androgen-mediated hair follicle miniaturization by finasteride for one month or more.

2. The novel long-acting injectable composition of claim 1, wherein the injectable composition is a subcutaneous (SC) injection formulation.

3. The novel long-acting injectable composition of claim 1, wherein a mean elimination half-life (t_{1/2el}) of finasteride *in vivo* following administration of the injectable composition is 100 to 160 hours.

4. The novel long-acting injectable composition of claim 1, wherein a first-order absorption rate constant (Ka) of finasteride following administration of the injectable composition is 0.0005 to 0.0015 h⁻¹.

5. The novel long-acting injectable composition of claim 1, wherein a volume of distribution of finasteride in central compartment (tvV) following administration of the injectable composition is 500 to 1,500 L.

6. The novel long-acting injectable composition of claim 1, wherein a volume of distribution of finasteride in peripheral compartment (tvV2) following administration of the injectable composition is 0.1 to 0.5 L.

7. The novel long-acting injectable composition of claim 1, wherein a clearance of finasteride from central compartment (tvCL) following administration of the injectable composition is 5 to 15 L/h.

8. The novel long-acting injectable composition of claim 1, wherein a clearance of finasteride between central compartment and peripheral compartment (tvCL2) following administration of the injectable composition is 0.005 to 0.015 L/h.

9. The novel long-acting injectable composition of claim 1, wherein a maximum plasma concentration (Cₘₐₓ) of finasteride following administration of the injectable composition is 500 to 3,000 pg/mL.

10. The novel long-acting injectable composition of claim 1, wherein a steady-state average concentration (C_{ss, avg}) of finasteride following administration of the injectable composition is 1,000 to 6,000 pg/mL.

11. The novel long-acting injectable composition of claim 1, wherein an AUC at 0 hour to 672 hours (ACU₀₋₆₇₂ₕ) of finasteride following administration of the injectable composition is 500,000 to 2,000,000 hr*pg/mL.

12. The novel long-acting injectable composition of claim 1, wherein a steady-state AUC (AUCss) of finasteride following administration of the injectable composition is 1,000,000 to 4,000,000 hr*pg/mL.
